# EUROPEAN PATENT APPLICATION

(11) **EP 3 919 052 A1**
(43) Date of publication of application: **08.12.2021**
(21) Application number: 20739000.6
(22) Date of filing: 09.01.2020
(51) Int. Cl.: A61K 31/137, A61K 31/403, A61K 31/407, A61K 31/47, A61P 27/02, A61K 9/06, A61K 9/08, A61K 9/20

(54) **PHARMACEUTICAL COMPOSITION FOR INTRAOCULAR OR ORAL ADMINISTRATION FOR TREATMENT OF RETINAL DISEASES**

(30) Priority: 09.01.2019 JP 2019001979
(71) Applicant: Fuso Pharmaceutical Industries, Ltd., Osaka-shi, Osaka 541-0045 (JP)
(72) Inventor: SHINTO, Saki, Fukuoka-shi, Fukuoka 812-8582 (JP); ISHIDA, Minato, Fukuoka-shi, Fukuoka 812-8582 (JP); YAMAMOTO, Keiichi, Osaka-shi, Osaka 536-8523 (JP); YAMADA, Ken-ichi, Fukuoka-shi, Fukuoka 812-8582 (JP)
(74) Representative: Boult Wade Tennant LLP
(86) International application number: PCT/JP2020/000538
(87) International publication number: WO 2020/145364

(57) **Abstract**

The present invention provides a pharmaceutical composition for intraocular administration or oral administration, for preventing or treating a retinal disease (such as age-related macular degeneration), or suppressing a progression of the disease in a subject in need of treatment, the composition comprising an effective amount of one or more compounds selected from the following group, and a pharmaceutically acceptable carrier; and a method threfor, a group consisting of apomorphine, eseroline, ethoxyquin, methyldopa, olanzapine and indapamide.

## Description

### TECHNICAL FIELD

The present invention provides pharmaceuticals and methods for intraocular or oral administration use for preventing or treating a retinal disease (such as age-related macular degeneration).

### BACKGROUND ART

Many retinal diseases, including retinitis pigmentosa and age-related macular degeneration, have unknown causes, and are intractable. Humans obtain external information through the five senses including vision, and in particular, 80% or more of the information is obtained from vision. Accordingly, visual impairment due to intractable retinal disease may reduce significantly the patient's quality of life (QOL).

Age-related macular degeneration (AMD) is known to be a disease with high unmet medical needs in which treatment satisfaction is low and contribution of drugs for the treatment is low. Age-related macular degeneration is roughly categorized based on pathogenic mechanism into two types, the atrophic (dry) and the exudative (wet). In the United States, among the age-related macular degeneration patients, atrophic (dry) patients account for a large proportion of about 85% to about 90%, while in Japan, exudative (wet) patients accounts for a large proportion of about 92%. However, effective therapeutic drugs for the atrophic (dry) disease have not been known. Also, regarding therapeutic drugs for the exudative (wet) type disease, only anti-vascular endothelial growth factor (abbreviated: anti-VEGF) drug by intravitreal injection administration has been known. Moreover, therapeutic drugs effective for treating or preventing of age-related macular degeneration, and suppressing progression of the disease have not been developed yet from substances having antioxidant property.

So far, some compounds having therapeutic activity against age-related macular degeneration have been reported, and are under development. For example, according to the patent document 1, Levodopa (another name, L-dopa (DOPA)), which is known as a therapeutic drug for Parkinson's disease, is reported to have a therapeutic activity against age-related macular degeneration.

Aso, many of the compounds under development as therapeutic drugs for age-related macular degeneration, including the above-mentioned anti-VEGF drug, are administered by an intravitreal injection administration. Also, although some cases by an administration through a local route or an oral administration have been reported, the dose thereof is not low. Accordingly, it is desired to develop an eye drop that can be suppressed its administration dose and also can be easily administered.

As the characteristics of the mechanism of the above-mentioned onset of the atrophic (dry) type of age-related macular degeneration, an event where an inflammation is occurred on retinal pigment epithelium cells (RPE cells) and thereby some photoreceptor cells are lost, so-called cell death is known as one of the causes. Here a retina is rich in unsaturated fatty acids (Non-patent document 1), and eyes are constantly exposed to light, and are known to consume a large amount of oxygen (Non-patent document 2). Accordngly, since it can be said that the retina is in an environment susceptible to oxidative damage, it can be expected to lead to unprecedented therapeutic drugs by developing antioxidative therapeutic drugs.

### CITATION LIST

### PATENT DOCUMENT

Patent Document 1: JP 2011-520788 A1

### NON-PATENT DOCUMENT

Non-Patent Document 1: Acar, N. et al., PLosS. One., 2012, 7, 35102
Non-Patent Document 2: Ye.X. et al., Trends. Mol. Med., 2010, 16, 417-25

### SUMMARY OF INVENTION

### (PROBLEMS TO BE SOLVED BY INVENTION)

The present invention provides pharmaceuticals and methods for use of intraocular administration (including eye drop, and eye ointment) or oral administration, for preventing or treating age-related macular degeneration, or suppressing a progression of the disease, by using certain compounds having antioxidative properties, in particular anti-lipid peroxidative properties.

### (MEANS TO SOLVE PROBLEMS)

The inventors found out that a pharmaceutical composition for use of intraocular administration (including eye drop, and eye ointment) or oral administration, the pharmaceutical composition comprising an effective amount of certain compounds and pharmaceutically acceptable carriers is useful for preventing or treating retinal diseases (such as age-related macular degeneration), or suppressing a progression of the disease.

That is, the present invention provides the following embodiments, but are not limited thereto.

### (Pharmaceutical Composition)

[1] A pharmaceutical composition for intraocular administration or oral administration, for preventing or treating a retinal disease, or suppressing a progression of the disease in a subject in need of treatment, the composition comprising an effective amount of one or more compounds selected from the following group, and a pharmaceutically acceptable carrier,
   a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).
[2] The pharmaceutical composition according to [1] wherein the compound is one or more compounds selected from the group consisting of the following group,
   a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, and indapamide (4-chloro-N-[(2RS)-2-methyl-2,3-dihydro-1H-indol-1-yl]-3-sulfamoyl benzamide).
   [2-2] The pharmaceutical composition according to [1] or [2] wherein the compound is one or more compounds selected from the group consisting of the following group,
   a group consisting of ethoxyquin (6-ethoxy-2,2,4-trimethyl-1,2-dihydroquinoline), and methyldopa (methyldopa sesquihydrate).
[3] The pharmaceutical composition according to [1] or [2] wherein the retinal disease is one or more disease selected from the group consisting of diabetic retinopathy, retinitis pigmentosa, Stargardt disease, hypertensive retinopathy, glaucoma, cataract, central retinal artery occlusion, polypoidal choroidal vasculopathy, Leber congenital amaurosis (LCA), Usher syndrome, choroideremia, rod-cone or cone-rod dystrophy, ciliary disorder, mitochondrial disease, progressive retinal atrophy, geographic atrophy, familial or acquired macular degeneration, retinal photoreceptor disease, disease on retinal pigment epithelium, cystoid macular edema, retinal degenerative disease, vitreous opacity, uveitis, retinal detachment, traumatic retinal damage, iatrogenic retinal injury, macular hole, macular telangiectasia, ganglion cell disease, optic nerve cell disease, optic neuropathy, ischemic retinal disease, retinopathy of prematurity, retinal vascular occlusion, familial macroaneurysm, retial vascular disease, ocular vascular disease, vascular disease, and ischemic optic neuropathy.
[4] The pharmaceutical composition according to any one of [1] to [3] wherein the retinal disease is an age-related macular degeneration.
[5] The pharmaceutical composition according to any one of [1] to [4] wherein the age-related macular degeneration is atrophic (dry) age-related macular degeneration.
[6] The pharmaceutical composition according to any one of [1] to [4] wherein the age-related macular degeneration is exudative (wet) age-related macular degeneration.
[7] The pharmaceutical composition according to any one of [1] to [6] which is formulated into eye drop or eye ointment.
   [7-2] The pharmaceutical composition according to any one of [1] to [6] which is formulated into eye drop, eye ointment, eye gel, eye cream, tablet, fine granule, or capsule.
[8] The pharmaceutical composition according to any one of [1] to [7] wherein an effective dose of the compound is a low dose of about one-fifth or less of the half-lethal dose of each compound.
   [8-2] The pharmaceutical composition according to any one of [1] to [7] wherein an effective dose of the compound is a low dose of about one-tenth or less of the half-lethal dose of each compound.
   [8-3] The pharmaceutical composition according to any one of [1] to [7] wherein an effective dose of the compound is a low dose of about one-twentyth or less of the half-lethal dose of each compound.
   [8-4] The pharmaceutical composition according to any one of [1] to [7] wherein an effective dose of the compound is a low dose of about one-fiftyth or less of the half-lethal dose of each compound.
[9] The pharmaceutical composition according to any one of [1] to [8] wherein an effective dose of the compound is about five(5) weight % or less based on the weight of the pharmaceutical composition for intraocular administration, and is about eighty(80) weight % or less based on the weight of the pharmaceutical composition for oral administration.
   [9-2] The pharmaceutical composition according to any one of [1] to [8] wherein an effective dose of the compound is about three (3) weight % or less based on the weight of the pharmaceutical composition for intraocular administration, and is about fifty(50) weight % or less based on the weight of the pharmaceutical composition for oral administration.
   [9-3] The pharmaceutical composition according to any one of [1] to [8] wherein an effective dose of the compound is about one(1)weight % or less based on the weight of the pharmaceutical composition for intraocular administration.
   [9-4] The pharmaceutical composition according to any one of [1] to [8] wherein an effective dose of the compound is about zero point five(0.5)weight % or less based on the weight of the pharmaceutical composition for intraocular administration.

### (Eye Drop)

[10] An eye drop for preventing or treating a retinal disease, or suppressing a progression of the disease in a subject in need of treatment, the eye drop comprising an effective amount of one or more compounds selected from the following group, and a pharmaceutically acceptable carrier,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).

[10-2] The eye drop according to [10] wherein the compound is one or more compounds selected from the group consisting of the following group,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno(2,3-b][1,5]benzodiazepine, and indapamide (4-chloro-N-[(2RS)-2-methyl-2,3-dihydro-1H-indol-1-yl]-3-sulfamoyl benzamide). [10-3] The eye drop according to [10] wherein the compound is one or more compounds selected from the group consisting of the following group,
a group consisting of ethoxyquin (6-ethoxy-2,2,4-trimethyl-1,2-dihydroquinoline), and methyldopa (methyldopa sesquihydrate).

[10-4] The eye drop according to [10] which is a sterilized type of unit dosage form not containing a preservative.

[10-5] The eye drop according to [10] which does not substantially contain benzalkonium chloride.

### (Oral Drug)

[11] An oral drug for preventing or treating a retinal disease, or suppressing a progression of the disease in a subject in need of treatment, the oral drug comprising an effective amount of one or more compounds selected from the following group, and a pharmaceutically acceptable carrier,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).

[11-2] The oral drug according to [11] wherein the compound is one or more compounds selected from the group consisting of the following group,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, and indapamide (4-chloro-N-[(2RS)-2-methyl-2,3-dihydro-1H-indol-1-yl]-3-sulfamoyl benzamide).

[11-3] The oral drug according to [11] wherein the compound is one or more compounds selected from the group consisting of the following group,
a group consisting of ethoxyquin (6-ethoxy-2,2,4-trimethyl-1,2-dihydroquinoline), and methyldopa (methyldopa sesquihydrate).

### (Use)

[12] Use of one or more compounds selected from the following group in preparation of a pharmaceutical for intraocular administration or oral administration for preventing or treating a retinal disease, or suppressing a progression of the disease in a subject in need of treatment,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).

[12-2] The use according to [12] wherein the compound is one or more compounds selected from the group consisting of the following group,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, and indapamide (4-chloro-N-[(2RS)-2-methyl-2,3-dihydro-1H-indol-1-yl]-3-sulfamoyl benzamide).

[12-3] The use according to [12] wherein the compound is one or more compounds selected from the group consisting of the following group,
a group consisting of ethoxyquin (6-ethoxy-2,2,4-trimethyl-1,2-dihydroquinoline), and methyldopa (methyldopa sesquihydrate).

### (Method)

[13] A method for preventing or treating a retinal disease, or suppressing a progression of the disease in a subject in need of treatment, which comprises administering an effective amount of one or more compounds selected from the following group to a subject in need of treatment, a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).

[13-2] The method according to [13] wherein the compound is one or more compounds selected from the group consisting of the following group,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, and indapamide (4-chloro-N-[(2RS)-2-methyl-2,3-dihydro-1H-indol-1-yl]-3-sulfamoyl benzamide).

[13-3] The method according to [13] wherein the compound is one or more compounds selected from the group consisting of the following group,
a group consisting of ethoxyquin (6-ethoxy-2,2,4-trimethyl-1,2-dihydroquinoline), and methyldopa (methyldopa sesquihydrate).

### (Others)

[14] The pharmaceutical composition according to [1], the eye drop according to [10], or the oral drug according to [11], which further comprises one or more compounds for increasing an efficacy of the pharmaceutical composition, the eye drop, or the oral drug.

[14-1] The pharmaceutical composition according to [1], the eye drop according to [10], or the oral drug according to [11], wherein the one or more compounds for increasing an efficacy of the pharmaceutical composition, the eye drop, or the oral drug is antibiotic, steroid, anti-inflammatory agent, analgesic, surfactant, chelating agent, buffering agent, pH adjusting agent, or adjuvant, or any combinations of two or more of these compounds.

[14-2] The pharmaceutical composition according to [1], the eye drop according to [10], or the oral drug according to [11], wherein the one or more compounds for increasing an efficacy of the pharmaceutical composition, the eye drop, or the oral drug is vitamin C source, vitamin E source, vitamin A source, beta-carotene, zinc source, or copper source, or any combinations of two or more of these compounds.

[15] The pharmaceutical composition according to [1], the eye drop according to [10], or the oral drug according to [11], wherein the subject in need of treatment is a mammalian.

[15-2] The pharmaceutical composition according to [1], the eye drop according to [10], or the oral drug according to [11], wherein the subject in need of treatment is a human.

[15-3] The pharmaceutical composition according to [1], the eye drop according to [10], or the oral drug according to [11], wherein the subject in need of treatment is a human having 60 or more ages.

[15-4] The pharmaceutical composition according to [1], the eye drop according to [10], or the oral drug according to [11], the use according to [12], or the method according to [13], wherein the subject in need of treatment is a human containing some Drusen deposits.

[15-5] The pharmaceutical composition according to [1], the eye drop according to [10], or the oral drug according to [11], the use according to [12], or the method according to [13], wherein the subject in need of treatment is white race.

[16] The pharmaceutical composition according to [1], the eye drop according to [10], or the oral drug according to [11], for use to prevent or treat a retinal disease, or suppress a progression of the disease in a subject in need of treatment.

[17] A food or beverage for preventing or treating a retinal disease, or suppressing a progression of the disease, the food or beverage comprising the above-mentioned compound according to [1].

[18] The food or beverage according to [17], which is a health food, a functional food, a food for specified health uses, a nutritional supplement, a food with a label of Reduction of Disease Risk Claims, or a food for the sick.

[19] A method for preventing or treating a disease due to an activation of glial cell of retina (for example, muller cell, microglia), or suppressing a progression of the disease, comprising administering a therapeutically effective amount of the above-mentioned compound according to [1].

[20] A method for preventing or treating a disease due to an increased expression amount of specific gene (for example, mRNA of marker protein) which exists in glial cell of retina (for example, muller cell, microglia), or suppressing a progression of the disease, comprising administering a therapeutically effective amount of the above-mentioned compound according to [1] .

[21] A method for preventing or treating a disease due to an activity of a pathway of a complement, or suppressing a progression of the disease, which comprises administering a therapeutically effective amount of the above-mentioned compound according to [1].

[22] A method for preventing or treating a disease due to an increased expression amount of mRNA of speciifc complement, or suppressing a progression of the disease, comprising administering a therapeutically effective amount of the above-mentioned compound according to [1].

### [Effect]

The pharmaceutical composition of the present invention for intraocular or oral admnistration comprising specifc compound having anti-lipid peroxidation property is useful for preventing or treating a retinal disease, for example, age-related macular degeneration (in particular, atrophic (dry) of age-related macular degeneration), or suppresing a progression of the disease.

### BRIEF DESCRIPTION OF DRAWINGS

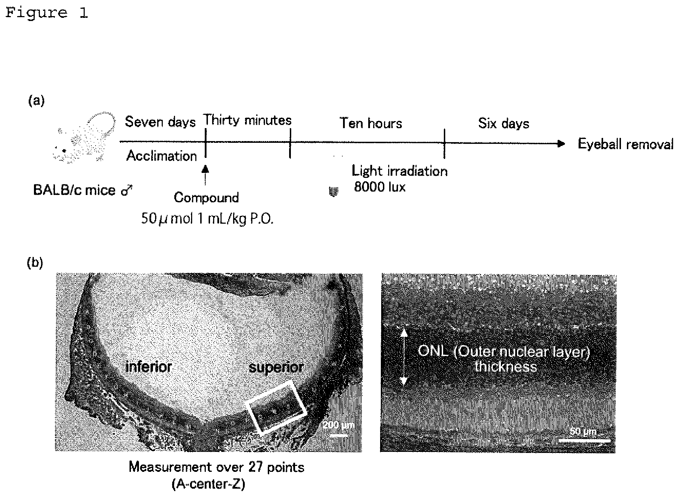

[Figure 1] Figure 1 is charts showing a method for producing age-related macular degeneration (AMD) model mice (production schedule) in the case of oral administration. (a) AMD model mouse production schedule; (b) ONL measurement method in which ONL thickness was measured over 27 points (A-center-Z) every 180 µm (left: observation field of 4 times, right: observation field of 60 times).

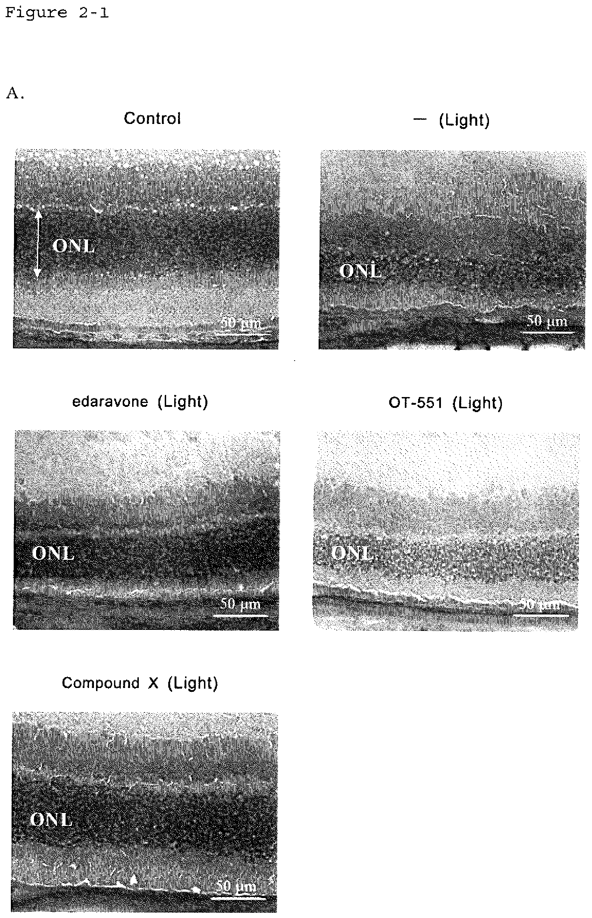

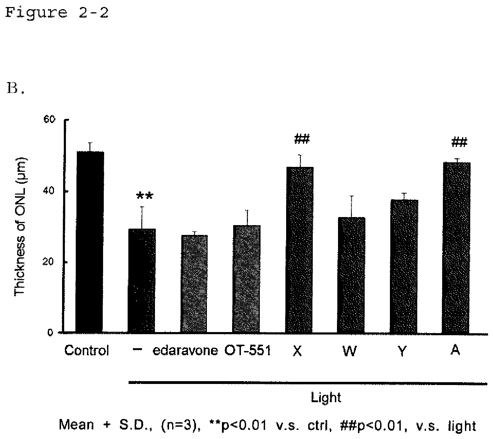

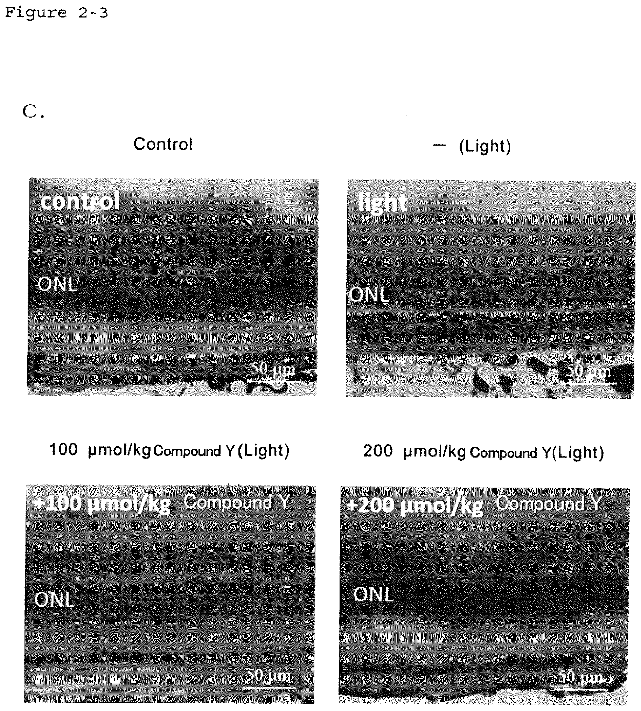

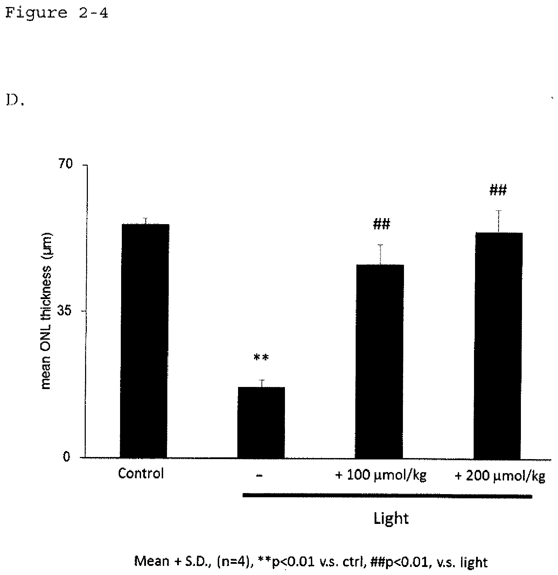

[Figure 2] Figure 2 (A) is charts showing the results of evaluating the thickness of an outer nuclear layer (ONL) in the case of oral administration, and also showing ONL bright field image (observation magnification: 60 times) in the case of the dose of 50 µmol/kg of the present compounds (ethoxyquin (Compound X), eseroline (Compound W), methyldopa (Compound Y), and indapamide (Compound A)) compared to Control or control comound (edaravone, OT-551). Figure 2 (B) is charts showing the results of evaluating the thickness of an outer nuclear layer (ONL) in the case of oral administration, and also showing mean thickness of an outer nuclear layer (ONL) (Mean + S.D., (n=3-5), mean+S.D., **p<0.01 v.s. cotrol, ## p<0.01 v.s. Light). Figure 2 (C) is charts showing the results of evaluating the thickness of an outer nuclear layer (ONL) in the case of oral administration, and also showing ONL bright field image (observation magnification: 40 times) in the case of the dose of 100 µmol/kg or 200 µmol/kg of the present compounds (methyldopa (Compound Y)) compared to Control. Figure 2 (D) is is charts showing the results of evaluating the thickness of an outer nuclear layer (ONL) in the case of oral administration, and also showing mean thickness of an outer nuclear layer (ONL) (n=4, **p<0.01 v.s. ctrl, ## p<0.01 v.s. light).

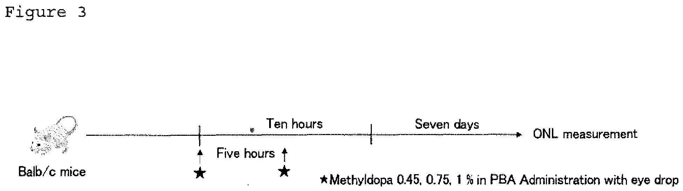

[Figure 3] Figure 3 is charts showing a method for producing age-related macular degeneration (AMD) model mice (production schedule) in the case of administration with eye drop.

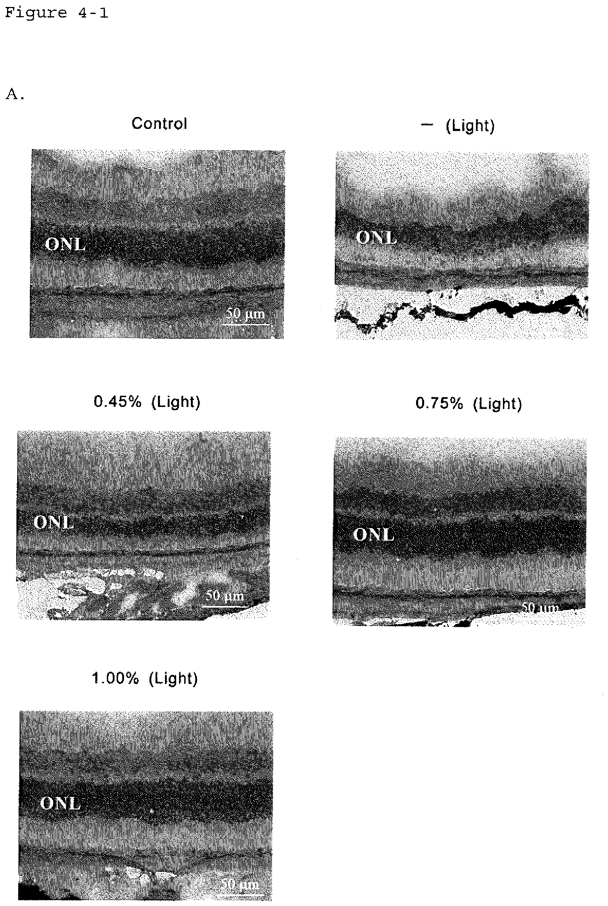

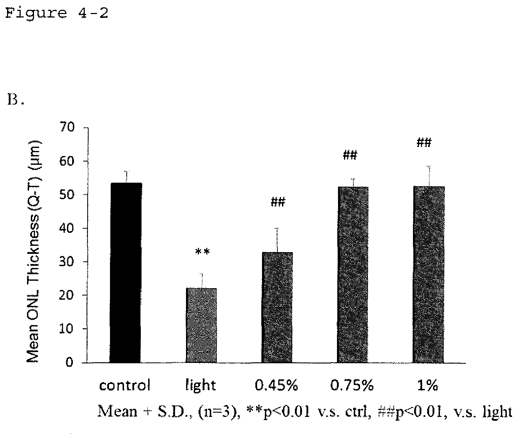

[Figure 4] Figure 4 (A) is charts showing the results of evaluating the thickness of an outer nuclear layer (ONL) in the case of administration with the eye drop of 0.45 %, 0.75 % or 1 % concentration of methyldopa and also showing ONL bright field image (observation magnificantion: 40 times) in the case of administration of 0.45 %, 0.75 % or 1 % concentration with the eye drop of the present compound (methyldopa (Compound Y)) compared to Control. Figure 4 (B) is charts showing the results of evaluating the thickness of an outer nuclear layer (ONL) in the case of oral administration, and also showing mean thickness of ONL of the present compound (methyldopa (Compound Y)) compared to Control (n=3, **p<0.01 v.s. ctrl, ## p<0.01 v.s. light).

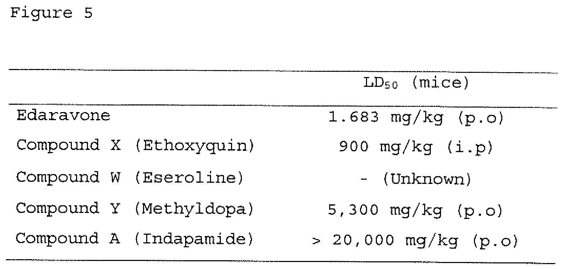

[Figure 5] Figure 5 is a table summarizing main action/action points, half-lethal dose (LD₅₀) and animal administration examples for the activity indicator compound and the present compound. The LD₅₀ was shown for edaravone and the four compounds used in this study. Here, oral represents oral administration, p.o. represents per os (oral administration), and i.p. represents intraperitoneal injection.

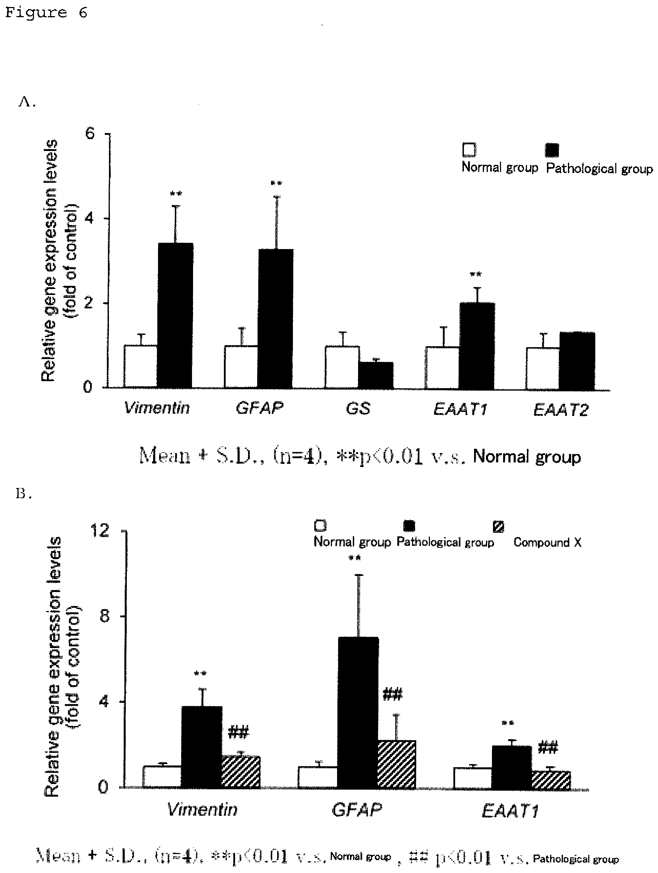

[Figure 6] Figure 6 is charts showing the results of activity evaluation test of Muller cell. Figure 6 (A) is charts showing a change of expression amounts of mRNA of muller cell marker protein in a normal group of mice and a pathological group of mice in the case of not administrating with any test compound. Also Figure 6 (B) is charts showing a change of expression amounts of mRNA of muller cell marker protein in a normal group of mice, a pathological group of mice in the case of not administrating with any test compound, and a pathological group of mice in the case of administrating with any test compound.

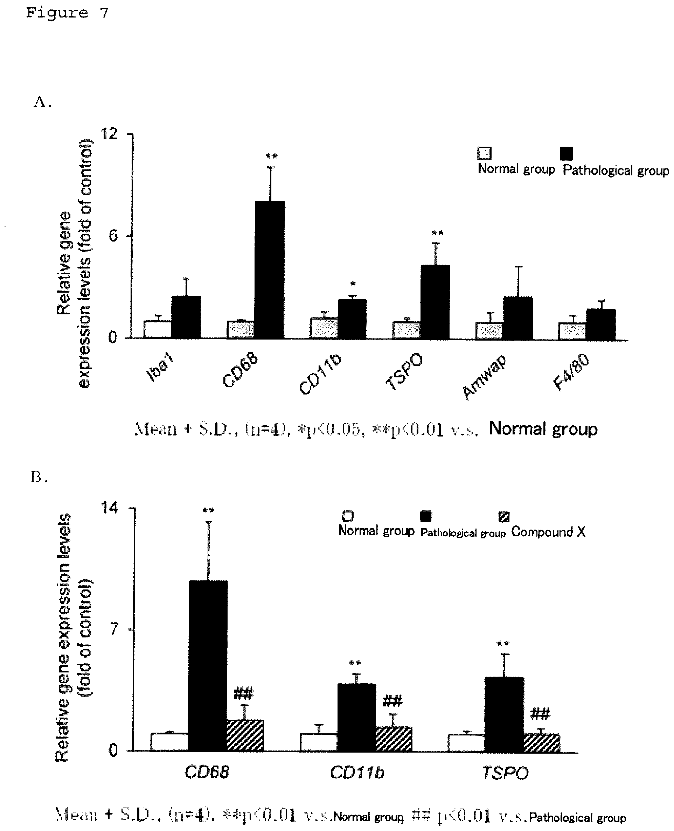

[Figure 7] Figure 7 is charts showing the results of activity evaluation test of microglia. Figure 7 (A) is charts showing a change of expression amounts of mRNA of microglia marker protein in a normal group of mice and a pathological group of mice in the case of not administrating with any test compound. Also Figure 7 (B) is charts showing a change of expression amounts of mRNA of microglia marker protein in a normal group of mice, a pathological group of mice in the case of not administrating with any test compound, and a pathological group of mice in the case of administrating with any test compound.
[Figure 8] Figure 8 is charts showing the results of activity evaluation test of the complement.
Figure 8 (A) is charts showing a change of expression amounts of mRNA of the complement in a normal group of mice and a pathological group of mice in the case of not administrating with any test compound. Also, Figure 8 (B) is charts showing a change of expression amounts of mRNA of the complement in a normal group of mice, a pathological group of mice in the case of not administrating with any test compound, and a pathological group of mice in the case of administrating with any test compound.

### MODE FOR CARRYING OUT THE INVENTION

### (Pharmaceutical Use)

The term of "retinal disease" represents diseases and disorders in retina, and specific examples thereof include one or more diseases selected from the group consisting of diabetic retinopathy, retinitis pigmentosa , Stargardt disease, hypertensive retinopathy, glaucoma, cataract, central retinal artery occlusion, polypoidal choroidal vasculopathy, Leber congenital amaurosis (LCA), Uscher syndrome, choroideremia, rod-cone or cone-rod dystrophy, ciliary disorder, mitochondrial diseases, progressive retinal atrophy, geographic atrophy, familial or acquired macular degeneration, retinal photoreceptor disease, diseases due to retinal pigment epithelium, cystoid macular edema, retinal degenerative disease, vitreous opacity, uveitis, retinal detachment, traumatic retinal damage, iatrogenic retinal injury, macular hole, macular telangiectasia, ganglion cell disease, optic nerve cell disease, optic neuropathy, ischemic retinal disease, retinopathy of prematurity, retinal vascular occlusion, familial macroaneurysm, retial vascular disease, ocular vascular disease, ocular vascular disease, or ischemic optic neuropathy. Preferred specific examples thereof include diabetic retinopathy, retinitis pigmentosa, and age-related macular degeneration.

The term of "age-related macular degeneration (AMD)" represents an aging-related disease causing a vidual loss of macula (center of field of view) due to a damage of retina. The age-related macular degeneration is started from Drusen (yellow deposits) in macula between retinal pigment epithelium and choroid under its epithelium. The subjects containing a drusen may develop following the symptom to those having age-related macular degeneration.

Examples of the age-related macular degeneration include atrophic (dry) type of disease and exudative (wet) type of disease, which are generally known. The development of the geographic atrophy may be observed as the characteristics of the atrophic (dry) type of age-related macular degeneration, while the development of angiogenesis may be observed as the characteristics of the exudative type of macular degeneration degeneration.

The age-related macular degeneration used herein encompasses related symptoms of age-related macular degeneration. Examples of the related symptoms include one or more symptoms including a blur due to distortion of the retina in center field of view (metamorphopsia); a loss of central area (central scotoma) and a reduction of overall eyesight (poor eyesight), due to a disorder of retina; a difficulty in distinguising a certain colour and another colour (color blindness); an obstruction of retinal pigment epithelium (RPE) (including aggregation and/or loss of pigment); RPE detachment; geographic atrophy; a subretinal neovascularization, a disc-shaped scar, and astigmatism (deformed vision), and aren't limited thereto.

As glial cells existed in a retina, a Muller cell, microglia or the others are known. When the retina is subjected to a lipid peroxidation, a Muller cell or micrglia as glial cell is activated. It has been presumed that inflammatory substances are secreted from the activated glial cells, and the complement pathway which is accompanied by the phenomenon is activated, and finally, cell dealth (deterioration of pathology) may be caused. Also, it is known that these glial cells are activated in retinal diseases (such as age-related macular degeneration), and an expression amount of marker protein by activation of the glial cells are increased in a pathological group. Accordingly, the suppression of the expression amount of these proteins can be used as an indicator for examining an effectiveness of a certain compound against a treatment, prevention or suppression of retinal diseases (such as age-related macular degeration).

In an experiment for analysis of glial cell activation of the present invention, a real-time PCR method may be used, but is not be limited to this method. Here the real-tme PCR method represents a method for monitoring the PCR amplification amounts in real-time and then analysing them. Specific examples of the real-time PCR method include generally-known methods, and may be carried out by using commercially available reagents (such as a primer, and a fluorescent substance) and devices.

As used herein, the "treatment or treating" or "prevention or preventing" or "suppression or suppressing of a progression" against retinal disease encompasses at least one of the followings. (1) excluding the above-mentioned retinal disease, for example, one or more of the above-mentioned specific diseases, typically the AMD disease, and one or more of the relevant symptoms; (2) reducing or minimizing the degree of seriousness of the above-mentioned retinal disease, for example, one or more of the above-mentioned specific diseases, typically the AMD disease and one or more of the relevant symptoms: (3) delaying the progression or the onset of the above-mentioned retinal disease, for example, one or more of the above-mentioned specific diseases, typically, the AMD disease and one or more of the relevant symptoms; and (4) lowering, minimizing or excluding an incidence or a frequency of the above-mentioned retinal disease, for example, one or more of the above-mentioned specific diseases, typically, AMD disease and one or more of the relevant symptoms.

The "subject" for the purpose of the treatment and so on used herein represents mammalian, and includes human or non-human animals, and preferably includes human. The subject encompasses human of ages of 60 ages or more (preferably 65 ages or more, and more preferably 70 ages or more), and also preferably human containing Drusen deposits. For example, the white race which is sensitive to the atrophic (dry) type of disease is more preferaly included.

The present invention provides a pharmaceutical composition as an intraocular administration use (including eye drop, and eye ointment) or an oral administration use, for preventing or treating retinal diseases, or suppressing a progression of the diseases in a subject in need of the treatment and so on, the composition comprising as an active drug a therapeutically effective amount of one or more of the compounds selected from the following group (hereinafter, the compound may be referred to as "active drug of the present invention", or "present compound") and a pharmaceutically acceptable carrier (hereinafter, the pharmaceutical composition may be referred to as "pharmaceutical composition of the present invention" or "present pharmaceutical composition"). Examples of the active drug include the following groups: a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).

Here, the compounds which are included in the above-mentioned compound group are compounds which are found to show any permeability of the blood-brain barrier, and also a lipid peroxidation suppression effect in PCT/JP 2018/025496 filed by the inventors and so on of the present application. The structural formulae of these compounds are indicated below.

Apomorphine: ((R)-(-)-Apomorphine hydrochloride):

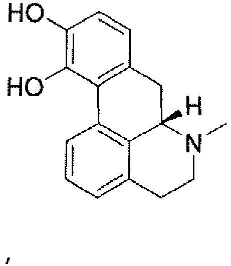

Eseroline: ((-)-Eseroline fumarate):

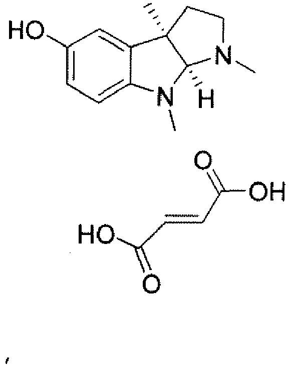

Ethoxyquin: (6-Ethoxy-2,2,4-trimethyl-1,2-dihydroquinoline):

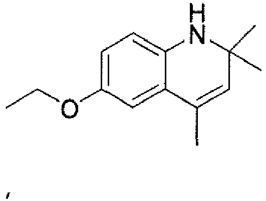

Methyldopa: (Methyldopa sesquihydrates):

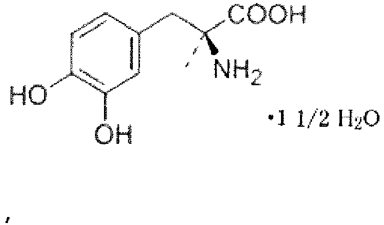

Olanzapine: (2-Methyl-4-(4-methyl-1-piperazinyl)-10H-thieno[2,3-b][1,5]benzodiazepine):

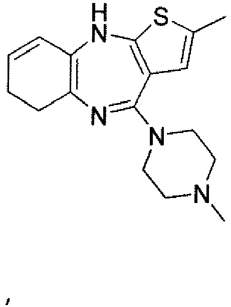

Methyl 3-amino-4-(phenylamino)benzoate:

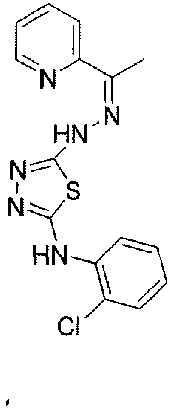

Methyl 3-amino-4-(benzylamino)benzoate:

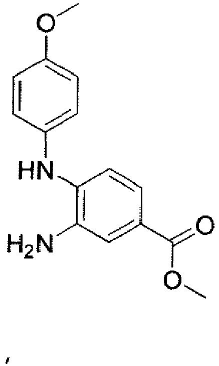

Methyl 3-amino-4-((1-phenylethyl)amino)benzoate:

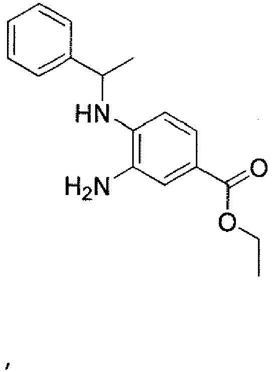

1-(4-(Trifluoromethoxy)phenyl)-1H-indol-5-amine:

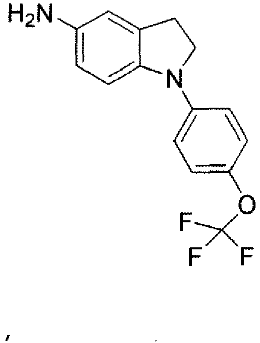

1-(3,5-Dimethylphenyl)-1H-indol-6-amine:

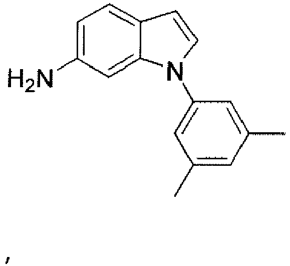

1-(3,5-Dimethylphenyl)indolin-6-amine:

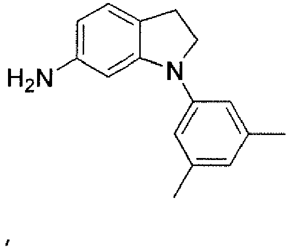

1-(4-Methoxyphenyl)-1H-indol-6-amine:

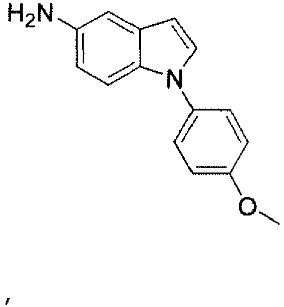

1-(4-(Methylthio)phenyl)-1H-indol-6-amine:

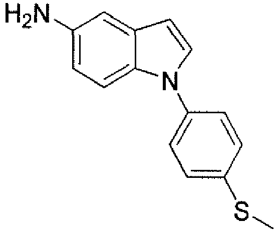

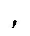

1-(4-(Trifluoromethoxy)phenyl)-1H-indol-5-amine:

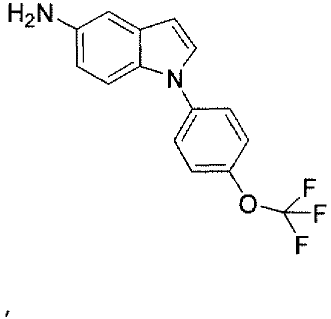

Indapamide: (4-Chloro-N-[(2RS)-2-methyl-2,3-dihydro-1H-indol-1-yl]-3-sulfamoyl benzamide):

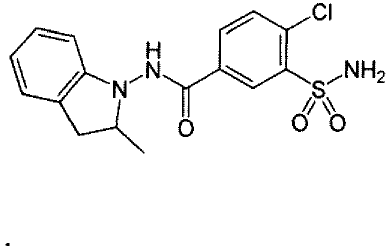

The present compound as active drug includes the forms of the above-mentioned compounds and pharmaceutically acceptable salts thereof. Also, the active drug of the present invention or a pharmaceutically acceptable salt thereof includes a hydrate or a solvate thereof with a solvent or the like. The present invention further includes any form of crystal of the active drug of the present invention.

Examples of the pharmaceutically acceptable salts include salts with organic bases (for example, diethanolamine salts, ethylenediamine salts), and salts with inorganic bases (for example, salts with alkali metals (for example, sodium, or potassium) and salts with alkaline earth metals (for example, calcium, or magnesium).

According to one embodiment, the compound as active drug is preferably one or more compounds selected from the following group: a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, and indapamide (4-chloro-N-[(2RS)-2-methyl-2,3-dihydro-1H-indol-1-yl]-3-sulfamoyl benzamide).

According to one embodiment, the compound as active drug is more preferably one or more compounds selected from the following group: a group consisting of ethoxyquin (6-ethoxy-2,2,4-trimethyl-1,2-dihydroquinoline), and methyldopa (methyldopa sesquihydrates).

As used herein, an "effective amount" represents an amount of an active drug which is sufficient to provide the desirable effect, that is, the treatment or prevention of the retinal disease (such as age-related macular degeneration), or the suppression of the progress of the diseases, which are as described herein. Also the active drug or the pharmaceutical composition of the present invention may be used in combination with a publicly known active drug or a pharmaceutical composition for the intended diseases or the relevant symptoms.

According to one embodiment, the effective amount of the present invention may be any dose as long as it is the dose capable of preventing an onset of the retinal disease (such as age-related macular degeneration), treating the symptoms of the diseases, or suppressing (improving) the progress of the symptoms of the disease when the present compound as active drug is administered. In the case of an oral administration, the dose may be an orally therapeutically effective amount, and in the case of an intraocular administration (such as eye drop or eye ointment), the dose may be an intraocularlly effective amount.

The low dose as effective amount represents a dose which is less than about one severalth to less than one a few tenth based on the half-lethal dose of each compound, when the present compound as an active drug is administered. For example, the low dose represents about one fifth, preferably about one tenth, more preferably about one twentyth, further preferably about one fiftyth based on the half-lethal dose of each compound, which is not be limited thereto.

Also, the dose range can be adjusted appropriately depending on a condition including a selection of the compound used, an administration route (for example, an oral administration or an intraocular administration), a property of a drug formulation, a type, age, weight or sex of a subject, or a property of a symptom or a degree of seriousness.

The effective amount of the present compound is about 5% by weight or less, more preferably about 3 % by weight or less, even more preferably about 1 % by weight or less, and further more preferably about 0.5 % by weight or less based on the weight of the pharmaceutical composition for intraocular administration, and is not limited thereto. Also the effective amount of the present compound is about 80% by weight or less, and more preferably about 50 % by weight or less based on the weight of the pharmaceutical composition for oral administration, and is not limited thereto.

Examples of the effective amount of the present compound include about 0.001 to about 50 mg/weight kg per a daily dose. For example, in the case of the oral administration, the daily dose is about 0.1 to about 50 mg/kg, usually about 0.2 to about 10 mg/kg, preferably about 0.3 to about 2 mg/kg, and more preferably about 0.4 to about 1 mg/kg, which is not limited thereto. For example, in the case of the intraocular administration, the daily dose is about 0.001 to about 0.1 mg/kg, usually about 0.01 to about 0.05 mg/kg, preferably about 0.02 to about 0.05 mg/kg, and more preferably about 0.03 to about 0.05 mg/kg, which is not limited thereto. In the case of administration with eye drop, typically about 0.5 mg/instillation per one-eye is administered in five to six divided times per day.

The present compound or the pharmaceutical composition of the present invention can be administered once per day, or a plural times per day (for example, in the case of the oral administration, they can be administered in two or three divided times per day, and in the case of the intraocular administration, they can be administered in two to ten divided times, for example, five to six divided times). Also they can be administered once or a plural times within several days or several weeks.

As used herein, the term of "administer (or administration)" represents that the present compound or the pharmaceutical composition of the present invention is provided or prescribed to an individual of subject, or the individual receives the present compound or the pharmaceutical composition of the present invention; and so on. The administration route of the present compound or the pharmaceutical composition may be either an oral administration route or an intraocular administration route, which can vary depending on intended disease or symptom, or age, weight or sex of the subject, or the like, and is preferably the intraocular administration (such as eye drop or eye ointment) in terms of an easy of administration and a lowering of dose and so on.

The pharmaceutical composition of the present invention can be administered by an oral administration route or an intraocular administration route to the subject.

The pharmaceutical composition of the present invention can be prepared as the pharmaceutical composition for oral administration or the pharmaceutical composition for intraocular administration by using conventionally publicly known technologies, and thereby can contain a non-toxic and inert carrier or additive (hereinafter, referred to as "pharmaceutically acceptable carrier") which is usually used in the pharmaceutical field. For example, the pharmaceutical composition for oral administration (oral formulations) is formulated into, for example, tablets, fine granules, capsules, pills, granules, powders, solutions, and suspensions and so on, which is not limited thereto. Also the pharamaceutical composition for intraocular administration is formulated into dosage forms such as eye drop, eye ointment, eye gel, eye cream, Tenon injectable, and ocular fundus injectable, and so on, but which is not limited to these forms, and eye drop or eye ointment is preferably included, and eye drop is more preferably included.

The "pharmaceutically acceptable carrier" used herein may be contained by combining various active ingredients or medicinal ingredients (including pharmacological active ingredients or physiologically active ingredients) or additives (for example, excipients, lubricants, binders, disintegrants, emulsifier, stabilizer, correctives, diluents, tonicity agents, buffer agents, pH adjusting agents, solubilizers, thickening agents, dispersing agents, preservatives (antiseptics)) in addition to the present compound as active drug depending on various kinds of usages such as the administration route and the dosage form as long as a pharmacological effect is not disturbed. These ingredients may be compounded appropriately within certain concentration ranges which do not affect any irritation and so on, and the kinds of the ingredients are not particularly limited thereto.

When the present compound or the pharmaceutical composition of the present invention is used as the pharmaceutical composition for oral administration, pharmaceutically acceptable carriers such as excipients, lubricants, binders, disintegrants, emulsifying agent, stabilizer, correctives, or diluents may be contained.

Examples of the excipients include organic excipients and inorganic excipients. Examples of organic excipients include one or more compounds selected from sugar derivatives (such as lactose, sucrose, glucose, mannitol, and sorbitol), and starch derivatives (such as corn starch, potato starch, α-starch, and dextrin), cellulose derivatives (such as crystalline cellulose), gum arabic, dextran, prolan and the others. Examples of the inorganic excipients include one or more compounds selected from light anhydrous silicic acid and sulfates (such as calcium sulfate).

Examples of the lubricants include one or more compounds selected from stearic acid, metal stearates (such as calcium stearate and magnesium stearate), talc, colloidal silica, waxes (such as bead wax), adipic acid, sulfates (such as sodium sulfate), glycol, fumaric acid, sodium benzoic acid, D,L-leucine, sodium lauryl sulfate, silic acids (such as silicic acid anhydride, silicic acid hydrate), and starch derivatives described as the above-mentioned excipients.

Examples of the binders include one or more compounds selected from hydroxypropyl cellulose, hydroxypropylmethyl cellulose, polyvinylpyrrolidone, macrogol, and compounds described as the above-mentioned excipients.

Examples of the disintegrants include one or more compounds selected from cellulose derivatives (such as low substituted hydroxypropyl cellulose, carboxymethyl cellulose, calcium carboxymethyl cellulose, and internally cross-linkedcarboxymethyl cellulose calcium), and chemically modified starch and cellulose derivatives (such as carboxymethyl starch, and carboxymethyl starch sodium).

Examples of the emulsifying agents include one or more compounds selected from colloidal clay (such as bentonite, and Veegum), anionic surfactants (such as sodium lauryl sulfate), cationic surfactants (such as benzalkonium chloride), and nonionic surfactants (such as polyoxyethylene alkyl ether, polyoxyethylene sorbitan fatty acid ester, and sucrose fatty acid ester).

Examples of the starbilizer include one or more compounds selected from p-hydroxybenzoic esters (such as methyl paraben, and propyl paraben), alcohols (such as chlorobutanol, benzylalcohol, and phenylethyl alcohol), phenols (such as phenol, and cresol), thimerosal, acetic anhydride, and sorbic acid.

Examples of the correctives include one or more compounds selected from sweetness (such as saccharin sodium, and aspartame), acidulants (such as citric acid, malic acid, and tartaric acid), and fragrances (such as menthol, and fruit extracts (such as orange extract)).

Examples of the diluents include one or more compounds selected from lactose, mannitol, glucose, sucrose, calcium sulfate, hydroxypropyl cellulose, microcrystalline cellulose, water, ethanol, polyethylene glycol, propylene glycol, glycerol, starch, and polyvinylpyrrolidone.

When the present compound or the pharmaceutical composition of the present invention is used as the pharmaceutical composition for intraocular administration, the composition may contain pharmaceutically acceptable carriers such as tonicity agents, buffer agents, pH adjusting agents, solubilizers, thickening agents, stabilizers, preservatives (antiseptics), and the like.

Examples of the tonicity agents include one or more compounds selected from glycerin, propylene glycol, sodium chloride, calcium chloride, sorbitol, and mannitol.

Examples of the buffer agents include one or more compounds selected from phosphoric acid, phosphate salt, citric acid, acetic acid, and ε-amino caproic acid.

Examples of the pH adjusting agents include one or more compounds selected from hydrochloric acid, citric acid, phosphoric acid, acetic acid, sodium hydroxide, calcium hydroxide, boric acid, borax, sodium hydrogen phosphate, sodium dihydrogen phosphate, sodium carbonate, and sodium hydrogencarbonate. The pH of the eye drop may be within a range which is acceptable as eye formulations, and preferably, it is usually adjusted to pH 4.0 to 8.5.

Examples of the solubilizers include one or more compounds selected from polysorbate 80, polyoxyethylene hardened castor oil 60, or macrogol 4000.

Examples of the thickening agents and dispersing agents include one or more compounds selected from cellulose polymer (such as hydroxypropyl methyl cellulose, and hydroxypropyl cellulose), polyvinyl alcohol, or polyvinylpyrrolidone.

Examples of the stabilizers include one or more compounds selected from edetic acid and sodium edetate.

Examples of the preservatives (antiseptics) include one or more compounds selected from sorbic acid, potassium sorbate, benzalkonium chloride, benzethonium chloride, methyl paraoxybenzoate, propyl paraoxybenzoate, or chlorobutanol.

The pharmaceutical composition, the eye drop, or the oral drug of the present invention may further contain one or more compounds for increasing efficacies. Examples of such a compound may include antibiotics, steroids, anti-inflammatory agents, analgesic agents, surfactants, chelating agents, buffer agents, pH adjusting agents, or adjuvants, or any combinations of two or more of them, and which is not be limited to these agents. Specific examples of the compounds in the case of eye drop may include vitamin C source, vitamin E source, vitamin A source, beta-carotene, zinc source, or copper source, or any combinations of two or more of these compounds, and which is not be limited to these agents.

According to one embodiment, the eye drop of the present invention is a sterilized type of unit dosage form not containing a preservative. Also according to one embodiment, the eye drop of the present invention is an eye drop which does not substantially contain benzalkonium chloride.

When the pharmaceutical composition of the present invention is used as an intraocular adminisitration formulation (such as eye drop), as needed, any compounds for increasing efficacies may be contained as long as an eye pain is caused, and examples of the compounds include anti-inflammatory drugs (such as epsilon aminocaproic acid, and diclofenac sodium), vasoconstrictor (such as tetrahydrozoline hydrochloride, and phenylephrine hydrochloride), antiallergic drug (such as sodium cromoglicate, and ketotifen fumarate), antihistamine drug (such as chlorpheniramine maleate, and diphenhydramine hydrochloride), or vitamins (such as riboflavin phosphate, cyanocobalamin, and panthenol), and which is not be limited to these compounds.

### EXAMPLES

The present invention is more specifically explained by the Formulation Examples and Test Examples as follows, and the present invention is not limited to these examples. The compounds, the mice, and the reagents which are used in the Examples were available from commercial sources, or were prepared according to publicly known methods.

### Formulation Examples

The Formulation examples of the pharmaceutical composition of the present invention are shown below, and are not limited thereto.

### (Formulation Example 1)

### Tablet

**[Table 1]**

| In 100 mg | |
|---|---|
| Methyldopa | 25 mg |
| Lactose | 49.5 mg |
| Corn starch | 11 mg |
| Carboxymethyl cellulose calcium | 7 mg |
| Hydroxypropyl cellulose | 7 mg |
| Magnesium stearate | 0.5 mg |

A tablet is prepared by a generally known formulating method of a tablet. Specifically, methyldopa as the present compound, corn starch, and lactose are mixed in a mixer, and carboxymethyl cellulose calcium, and hydroxypropyl cellulose are added to the mixture, and the mixture is granulated, and the resulting granules are subjected to a grain size procedure after drying, and magnesium stearate is added to the granules with grained size, and mixed with each other, and the mixture is compressed with a tableting machine. Also by changing an addition amount of the present compound, certain tablets containing a desirable amount of the present compound (such as 10 mg, 25 mg, or 50 mg) in a 100 mg tablet can be prepared.

### (Formulation Example 2)

### Eye Drop

**[Table 2]**

| In 100 mL | |
|---|---|
| Methyldopa | 500 mg |
| Sodium chloride | 1000 mg |
| Polysorbate 80 | appropriate |
| Sodium hydrogen phosphate | appropriate |
| Sodium dihydrogen phosphate | appropriate |
| Sterile purified water | appropriate |

An eye drop is prepared by a generally known formulating method of an eye drop. Specifically, into a sterile purified water, methyldopa as the present compound, and the above-mentioned ingredients other than methyldopa are added, and the mixture is mixed well to prepare a solution for eye drop. By changing an addition amount of the present compound such as methyldopa, certain eye drops containing a desirable amount of the present compound (such as 0.3 % (W/V), 0.5 % (W/V), or 1 % (W/V)) in a 100 mL eye drop can be prepared.

### (Formulation Example 3)

### Eye Ointment

**[Table 3]**

| In 100 g | |
|---|---|
| Methyldopa | 0.3g |
| Liquid paraffin | 10.0 g |
| white petrolatum | Appropriate |

An eye ointment is prepared by a generally known formulating method of an eye ointment. Specifically, into white petrolatum and liquid paraffin which are homogeneously melted, methyldopa as the present compound is added, and the mixture is mixed well, and afterthat, the resulting mixture is cooled gradually to prepare an eye ointment. By changing an addition amount of the present compound such as methyldopa, certain eye intments containing a desirable cncetration of the present compound (such as 0.3 % (W/W), 0.5 % (W/W), or 1 % (W/W)) in a 100 g eye ointment can be prepared.

### (Test Example 1)

### Pharmacological Test against age-related macular degeneration (AMD) (Oral administration)

The pharmacological activity (AMD) of the test compound against age-related macular degeneration (AMD) was examined. The test compounds are indicated below.

ethoxyquin (Compound X), eseroline (Compound W), methyldopa (Compound Y), and indapamide (Compound A).

A test was performed using a light irradiation model, which is widely employed as an atrophic AMD model mouse, as a test model.

### (Procedures)

First, AMD model mice were produced according to the following schedule.

### Laboratory animals

Male BALB/c mice (4 weeks old) were purchased from Japan SLC, Inc., and allowed to acclimate for a week before being used for experiments. Laboratory animal chow (CLEA Rodent Diet CE-2, CREA JAPAN, INC.) was used as food, and tap water was freely consumed as drinking water. The animals were raised under light and dark cycles of every 12 hours. Also all animal experiments were conducted under the approval of the Kyushu University Animal Experiment Committee.

### Production of light-induced AMD model mice

A method for producing age-related macular degeneration (AMD) model mice (production schedule) is shown in Figure 1.

To BALB/c mice, 1 mL/kg of 50 µM compound dissolved in PBS containing 10% polyethylene glycol (PEG) 300 was orally administered. Thirty (30) minutes later, one drop of Midrin P (5 mg/mL tropicamide, 5 mg/mL phenylephrine hydrochloride; Santen Pharmaceutical Co., Ltd.) was applied to each eye as a mydriatic drug. The mice were irradiated with 8000 lux white light for 10 hours, then returned to under normal light and dark cycles, and raised for 6 days. On day 7, the animal was euthanized by cervical dislocation, and the eyeballs were removed (Figure (1a)).

### Preparation of frozen eyeball section

Frozen sections of 8 µm thickness were prepared by using the above-mentioned removed eyeballs, and subjected to hematoxylin eosin (HE) staining, and the thickness of the outer nuclear layer (ONL) was measured over 27 points every 180 µm (observation magnification: 60 times) (Figure 1 (b)).

### HE staining

The preparation was air-dried for 1 hour, fixed with acetone at room temperature for 15 minutes, then immersed in 99.5% EtOH, 80% EtOH, 70% EtOH, and purified water in this order for 3 minutes each, and stained with hematoxylin for 10 minutes. Then, it was washed with running water for 10 minutes, soaked in warm water for 1 minute, and stained with eosin for 1.5 minutes. After washing with purified water, it was immersed in 70% EtOH, 80% EtOH, and 99.5% EtOH in this order for 3 minutes each, then washed with xylene three times, dried, and then enclosed with VectaMount (TM) Mounting Medium. The resultant was subjected to observation and imaging with Keyence fluorescence microscope (BZ-9000).

### Statistical analysis

Results were expressed as mean+standard deviation. Dunnett's Test was used for multigroup comparison.

### (Results)

The imaging results in which the thickness of an outer nuclear layer was evaluated are shown in Figure 2. In the case of the dose of 50 µmol/kg, the results of the ONL bright field image (observation magnification: 60 times) (Figure 2(a)) and the mean thickness of the ONL (Figure 2(b)) of the present compounds (ethoxyquin (Compound X), eseroline (Compound W), methyldopa (Compound Y), and indapamide (Compound A)), compared to a control and control compounds (Edaravone, OT-551 (4-cyclopropylcarboxy-1-hydroxy-2,2,6,6-tetramethyl-pyperidine) were obtained. The Figure 2(a) includes an inner nuclear layer (INL) in the top, an outer nuclear layer (ONL) in the middle, and a retinal pigment epithelium (RPE) in the bottom. When cell death occurs due to lipid peroxidation, the thickness of the outer nuclear layer in the middle becomes thinner.

First, the thickness of ONL was significantly reduced by light irradiation (Figure 2(a) negative control). The extent of ONL disorders was particularly severe on the superior side of the eyeball, and these results were consistent with the results known from the literature (see, for example, Tanito M., et al., Invest. Ophthalmol. Vis. SCI., 2007, 48 (4), 1900-5.).

On the other hand, in the case of the four present compounds used in this study, the ONL thickness in either case did not differ so much compared to that of the positive control. Significant thicknesses were observed compared to that of Edaravone and OT-551 as control compounds even at the same dose of 50 µmol/kg, and in particular, in the case of ethoxyquin (Compound X) and indapamide (Compound A), the thickness that was not different so much compared to that of the positive control was observed (Figure 2(b)).

Further, in the case of the dose of 100 µmol/kg or 200 µmol/kg, the results of the ONL bright field image (observation magnification: 40 times) (Figure 2(c)) and the mean thickness of the ONL (Figure 2(d)) of the present compound (methyldopa (Compound Y)), compared to a control were obtained. The thickness of the ONL of this case didn't have significant difference compared to that of the positive control (Figure 2(c)). Also the thickness that was not different so much as compared to that of the positive control was observed (Figure 2(d)).

For OT-551, which is a compound known to have a high retinal protective effect, approximately 100 mg/kg (360 µmol/kg) of the compound has been reported to be required in a light irradiation model mouse. Thus, the dose 50 µmol/kg in this study, about one-seventh of that of OT-551, is a considerably low dose.

In addition, 50 µmol/kg is less than one-tenth of each median lethal dose (LD50) of the five test compounds, thus the compounds have been confirmed to be safe (see Figure 5).

### (Test Example 2)

### Pharmacological Test against age-related macular degeneration (AMD) (Administration by eye drop)

The pharmacological activity of the test compound against age-related macular degeneration (AMD) was examined. The present compound as a test compound was methydopa (Compound Y) .

A test was performed using a light irradiation model, which is widely employed as an atrophic AMD model mouse, as a test model.

### (Procedures)

First, AMD model mice were produced according to the following schedule.

### Laboratory animals

Male BALB/c mice (4 weeks old) were purchased from Japan SLC, Inc., and allowed to acclimate for a week before being used for experiments. Laboratory animal chow (CLEA Rodent Diet CE-2, CREA JAPAN, INC.) was used as food, and tap water was freely consumed as drinking water. The animals were raised under light and dark cycles of every 12 hours. Also all animal experiments were conducted under the approval of the Kyushu University Animal Experiment Committee.

### Production of light-induced AMD model mice

A method for producing age-related macular degeneration (AMD) model mice (production schedule) is shown in Figure 3.

To BALB/c mice, Midrin P (5 mg/mL tropicamide, 5 mg/mL phenylephrine hydrochloride; Santen Pharmaceutical Co., Ltd.) as a mydriatic drug was applied to both eyes by one drop per one eye, and after 10 minutes, 0.45 %, 0.75 % or 1 % of methyldopa that was dissolved in PBS containing 10 % polyethylene glycol (PEG) 300 was administered with eye drop in 50 µL of the compound per one eye. Thereafter, the mice were irradiated with 8000 lux white light for 10 hours, then returned to under normal light and dark cycles, and raised for 6 days. On day 7, the animal was euthanized by cervical dislocation, and the eyeballs were removed.

### Preparation of frozen eyeball section

Frozen sections of 8 µm thickness were prepared by using the above-mentioned removed eyeballs, and subjected to hematoxylin eosin (HE) staining, and the thickness of the outer nuclear layer (ONL) was measured over 27 points every 180 µm (observation magnification: 40 times).

### HE staining

The preparation was air-dried for 1 hour, fixed with acetone at room temperature for 15 minutes, then immersed in 99.5% EtOH, 80% EtOH, 70% EtOH, and purified water in this order for 3 minutes each, and stained with hematoxylin for 10 minutes. Then, it was washed with running water for 10 minutes, soaked in warm water for 1 minute, and stained with eosin for 1.5 minutes. After washing with purified water, it was immersed in 70% EtOH, 80% EtOH, and 99.5% EtOH in this order for 3 minutes each, then washed with xylene three times, dried, and then enclosed with VectaMount (TM) Mounting Medium. The resultant was subjected to observation and imaging with Keyence fluorescence microscope (BZ-9000).

### Statistical analysis

Results were expressed as mean+standard deviation. Dunnett's Test was used for multigroup comparison.

### (Results)

The imaging results in which the thickness of an outer nuclear layer was evaluated are shown in Figure 4. The results of the ONL bright field image (observation magnification: 40 times) (Figure 4(a)) and the mean thickness of the ONL (Figure 4(b)) of the present compound (methyldopa (Compound Y)) compared to a control were obtained. The Figure 4(a) includes an inner nuclear layer (INL) in the top, an outer nuclear layer (ONL) in the middle, and a retinal pigment epithelium (RPE) in the bottom. When cell death occurs due to lipid peroxidation, the thickness of the outer nuclear layer in the middle becomes thinner.

First, the thickness of ONL was significantly reduced by light irradiation (Figure 4(a) negative control). The extent of ONL disorders was particularly severe on the superior side of the eyeball, and these results were consistent with the results known from the literature (see, for example, Tanito M., et al., Invest. Ophthalmol. Vis. SCI., 2007, 48 (4), 1900-5.).

On the other hand, in the case of methyldopa as the present compound used in this study, when the concentration of the compound in eye drop was 0.45 %, 0.75 % or 1 %, the ONL thickness in either case had significant thickness compared to that of the light irradiation case, and in particular, when the concentration of the compound was 0.75 % or more (0.75 % and 1 %), significant difference due to the difference of the concentration was not observed (Figure 4(b)).

Here the dose of the present compounds in the case of administration with the eye drop used in Test Example 2 described herein is a considerably low dose compared as the dose of the test compound in the case of oral administration used in Test Example 1 described herein. Accordingly, the dose of this case was confirmed to be remarkably low compared to the above-mentioned half lethal dose of the present compounds, and the present compounds have been thus confirmed to be safe.

From the above results, it is suggested that the present compound or the pharmaceutical composition of the present invention shows superior therapeutic activity against age-related macular degeneration in the case of intraocular administration (in partiuclar, eye drop) or oral administration, in particular intraocular administration (in partiuclar, eye drop).

### (Test Example 3)

### Activity evaluation test of Muller cell

For age-related macular degeneration (AMD), the suppression activity of the test compounds against the expression amount of mRNA of a marker protein, which is used as an indicator of activity of Muller cell and is increased as a pathology is deteriorated, was examined. The present compound as test compound was methyldopa (Compound Y).

### (Procedures)

First, AMD model mice were produced according to the following schedule.

### Laboratory animals

Male BALB/c mice (4 weeks old) were purchased from Japan SLC, Inc., and allowed to acclimate for a week before being used for experiments. Laboratory animal chow (CLEA Rodent Diet CE-2, CREA JAPAN, INC.) was used as food, and tap water was freely consumed as drinking water. The animals were raised under light and dark cycles of every 12 hours. Also all animal experiments were conducted under the approval of the Kyushu University Animal Experiment Committee.

### Production of light-induced AMD model mice

A method for producing age-related macular degeneration (AMD) model mice (production schedule) was prepared according to the method described in Test Example 2 (Figure 3) .

Specifically, to 9 week olds of BALB/c mice, 1 mL/kg of 50 µM test compounds dissolved in PBS containing 10% polyethylene glycol (PEG) 300 was orally administered. A PBS solution containing 10% polyethylene glycol (PEG) 300 was used as a control. Thirty (30) minutes later, one drop of Midrin P (5 mg/mL tropicamide, 5 mg/mL phenylephrine hydrochloride; Santen Pharmaceutical Co., Ltd.) as a mydriatic drug was applied to both eyes by one drop per one eye. The mice were irradiated with 8000 lux white light for 10 hours, then returned to under normal light and dark cycles, and raised for the desirable observation periods. After the observation periods, the animal was euthanized by cervical dislocation, and the retina were removed.

### Real-time PCR method

For the removed retina, RNA was extracted by using ISOSPIN Cell & Tissue RNA (manufactured by Nippon Gene Co. Ltd), and cDNA was then synthesized by using ReverTra Ace (TM) qPCR RT master Mix (manufactured by Toyobo Co. Ltd). The obtained cDNA was diluted with sterile water to 0.5 ng/*µ*L, and a real-time PCR was carried out with THUNDERBIRD (TM) SYBR (TM) qPCR Mix (manufactured by Toyobo Co. Ltd). All of the test results were corrected by Gapdh mRNA amounts, and a relative quantification was carried. The primer sequences that were used for each gene are shown in Table 4 below.

### (Results)

The test results are shown in Figure 6. It is shown in Figure 6A a change of expression amounts of mRNA of Muller cell marker protein in a normal group of mice and a pathological group of mice in the case of not administrating with any test compound. It is shown in Figure 6B a change of expression amounts of mRNA of Muller cell marker protein in a normal group of mice, a pathological group of mice in the case of not administrating with any test compound, and a pathological group of mice in the case of administrating with any test compound.

As shown in Figure 6A, the mice of the pathological group showed an increase in expression amounts of mRNA compared to the mice of the normal group. On the other hand, as shown in Figure 6B, when test compounds were administered to the mice in the pathological group, it was observed the remarkable suppression of the mRNA expression amounts wherein the remarkable increase in expression amounts of mRNA was observed in Figure 6A.

From these results, it was found that methyldopa as the present compound can suppress significantly the increase in expression amounts of mRNA in Muller cell marker protein which is related to age-related macular degeneration.

### (Test Example 4)

### Activity evaluation test of Microglia

For age-related macular degeneration (AMD), the suppression activity of the test compounds against the expression amount of mRNA of a marker protein, which is used as an indicator of activity of Muller cell and is increased as a pathology is deteriorated, was examined. The present compound as test compound was methyldopa (Compound Y).

### (Procedures)

First, experimental procedures for the production process of the test model mice, and the real-time PCR method were carried out by the method described in Test Example 3.

### (Results)

The test results are shown in Figure 7. It is shown in Figure 7A a change of expression amounts of mRNA of microglia marker protein in a normal group of mice, and a pathological group of mice in the case of not administrating with any test compound. It is shown in Figure 7B a change of expression amounts of mRNA of microglia marker protein in a normal group of mice, a pathological group of mice in the case of not administrating with any test compound, and a pathological group of mice in the case of administrating with any test compound.

As shown in Figure 7A, the mice of the pathological group showed an increase in expression amounts of mRNA compared to the mice of the normal group. On the other hand, as shown in Figure 7B, when test compounds were administered to the mice in the pathological group, it was observed the remarkable suppression of the mRNA expression amounts wherein the remarkable increase in expression amounts of mRNA was observed in Figure 7A.

From these results, it was found that methyldopa as the present compound can suppress significantly the increase in expression amounts of mRNA in microglia marker protein which is related to age-related macular degeneration.

The sequences of the primer that were used in the real-time PCR method that was carried in the Test Examples as described herein are shown in Table 4.
Table 4. Sequence of Primer used in Test Examples as described herein

**[Table 4]**

| Name | Primer | Sequence | SEQ ID No. |
|---|---|---|---|
| Vimentin | Forward | 5-CCTTGACATTGAGATTGCCA-3' | SEQ ID No. 22 |
| | Reverse | 5'-GTATCAACCAGAGGGAGTGA-3' | SEQ ID No. 23 |
| GFAP | Forward | 5'-AAGACACTGTGGCTCGTC-3' | SEQ ID No. 24 |
| | Reverse | 5'-CTTCCTGTAGGTGGCAAT-3' | SEQ ID No. 25 |
| GS | Forward | 5'-CCAACAAGCTGGTGCTATGTGAA-3' | SEQ ID No. 26 |
| | Reverse | 5'-CTGGTTGCTCACCATGTCCATTA-3' | SEQ ID No. 27 |
| EAAT1 | Forward | 5'-AATGTGGTATGCGCCTCTGG-3' | SEQ ID No. 28 |
| | Reverse | 5'-GCAGCAACCCTCCAATGAAA-3' | SEQ ID No. 29 |
| EAAT2 | Forward | 5'-GTGGCACCTCCATCTGAGGA-3' | SEQ ID No. 30 |
| | Reverse | 5'-CACCATCAGCTTGGCCTGTT-3' | SEQ ID No. 10 |
| Iba1 | Forward | 5'-GTCCTTGAAGCGAATGCTGG-3' | SEQ ID No. 11 |
| | Reverse | 5'-CATTCTCAAGATGGCAGATC-3' | SEQ ID No. 12 |
| CD68 | Forward | 5'-CTCTCTAAGGCTACAGGCTGCT-3' | SEQ ID No. 13 |
| | Reverse | 5'-TCACGGTTGCAAGAGAAACA-3' | SEQ ID No. 14 |
| CD11b | Forward | 5'-GTGTGACTACAGCACAAGCCG-3' | SEQ ID No. 15 |
| | Reverse | 5'-CCCAAGGACATATTCACAGCCT-3' | SEQ ID No. 16 |
| TSPO | Forward | 5'-TGCAGAAACCCTCTTGGCATC-3' | SEQ ID No. 17 |
| | Reverse | 5'-TGAAACCTCCCAGCTCTTTCC-3' | SEQ ID No. 18 |
| Amwap | Forward | 5'-TTTGATCACTGTGGGGATGA-3' | SEQ ID No. 19 |
| | Reverse | 5'-ACACTTTCTGGTGAAGGCTTG-3' | SEQ ID No. 20 |
| F4/80 | Forward | 5'-TGGAAGTGGATGGCATAGATG-3' | SEQ ID No. 21 |
| | Reverse | 5'-TTCACTGTCTGCTCAACG-3' | SEQ ID No. 22 |
| cls | Forward | 5'-CCCTGTAGCCACTTCTGCAA-3' | SEQ ID No. 23 |
| | Reverse | 5'-GGGCAGTGAACACA TCTCCA-3' | SEQ ID No. 24 |
| c4 | Forward | 5'-TCTGAAGCCTCCAACGTTCC-3' | SEQ ID No. 25 |
| | Reverse | 5'-TGGGATGGGGAAGGAAATGC-3' | SEQ ID No. 26 |
| cfb | Forward | 5'-GAAACCCTGTCACTGTCATTC-3' | SEQ ID No. 27 |
| | Reverse | 5'-CCCCAAACACATACACATCC-3' | SEQ ID No. 28 |
| cfd | Forward | 5'-CTGGGAGCGGCTGTATGT-3' | SEQ ID No. 29 |
| | Reverse | 5'-CACGGAAGCCATGTAGGG-3' | SEQ ID No. 30 |
| c3 | Forward | 5'-AGCCCAACACCAGCTACATC-3' | SEQ ID No. 31 |
| | Reverse | 5'-GAATGCCCCAAGTTCTTCGC-3' | SEQ ID No. 32 |
| c5 | Forward | 5'-CAGCAAGGAGGAGTCAACAT-3' | SEQ ID No. 33 |
| | Reverse | 5'-TCCACAAGAGCCCGTAAATC-3' | SEQ ID No. 34 |
| c9 | Forward | 5'-TGGTGACAACGACTGTGGAG-3' | SEQ ID No. 35 |
| | Reverse | 5'-TGTTGATCCCATAGCCTGCTG-3' | SEQ ID No. 36 |
| cfh | Forward | 5'-GTGGGCATCCCGGAGAC-3' | SEQ ID No. 37 |
| | Reverse | 5'- CACCAAACTCAAATTGAGATCCAA-3' | SEQ ID No. 38 |
| Gapdh | Forward | 5'-AATGTGTCCGTCGTGGATCTGA-3' | SEQ ID No. 39 |
| | Reverse | 5'-GATGCCTGCTTCACCACCTTCT-3' | SEQ ID No. 40 |

### (Test Example 5)

### Activity evaluation test of Complement

For age-related macular degeneration (AMD), the suppression activity of the test compounds against the expression amount of mRNA of a complement, which is increased as a pathology is deteriorated, was examined. The present compound as test compound was methyldopa (Compound Y).

### (Procedures)

Experimental procedures for the production process for the test model mice, and the real-time PCR method were carried out by the method described in Test Example 3.

### (Results)

The test results are shown in Figure 8. It is shown in Figure 8A a change of expression amounts of mRNA of a complement in a normal group of mice, and a pathological group of mice in the case of not administrating with any test compound. It is shown in Figure 8B a change of expression amounts of mRNA of a complement in a normal group of mice, a pathological group of mice in the case of not administrating with any test compound, and a pathological group of mice in the case of administrating with any test compound.

As shown in Figure 8A, the mice of the pathological group showed an increase in expression amounts of mRNA compared to the mice of the normal group. On the other hand, as shown in Figure 8B, when test compounds were administered to the mice in the pathological group, it was observed the remarkable suppression of the mRNA expression amounts wherein the remarkable increase in expression amounts of mRNA was observed in Figure 8A.

From these results, it was found that methyldopa as the present compound can suppress significantly the increase in expression amounts of mRNA in the complement which is related to age-related macular degeneration.

### [Industrial Applicability]

The present compound or the pharmaceutical composition of the present invention is useful as pharmaceuticals for an intraocular administration or an oral administration for treatment and the like of retinal diseases (such as age-related macular degeneration).

### [Free Text of Sequence Listing]

SEQ ID NOs: 1 to 40 each represents a PCR primer.

## Claims

1. A pharmaceutical composition for intraocular administration or oral administration, for preventing or treating a retinal disease, or suppressing a progression of the disease in a subject in need of treatment, the composition comprising an effective amount of one or more compounds selected from the following group, and a pharmaceutically acceptable carrier,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-i-piperazinyl)-10H-thieno[2,3-b][1,5]benzodiazepine, methyl 3-amino-4-(phenylamino)benzoate, methyl 3-amino-4-((4-methoxyphenyl)amino)benzoate, methyl 3-amino-4-((3-methoxyphenyl)amino)benzoate, methyl 3-amino-4-(benzylamino)benzoate, methyl 3-amino-4-((1-phenylethyl)amino)benzoate, 1-(4-(trifluoromethoxy)phenyl)indolin-5-amine, 1-(3,5-dimethylphenyl)-1H-indol-6-amine, 1-(3,5-dimethylphenyl)indolin-6-amine, 1-(4-methoxyphenyl)-1H-indol-6-amine, 1-(4-(methylthio)phenyl)-1H-indol-6-amine, 1-(4-(trifluoromethoxy)phenyl)-1H-indol-5-amine, and indapamide (4-chloro-N-[(2RS)-2-methyl-2,3-dihydro-1H-indol-1-yl]-3-sulfamoyl benzamide).

2. The pharmaceutical composition according to claim 1 wherein the compound is one or more compounds selected from the group consisting of the following group,
a group consisting of apomorphine ((R)-(-)-apomorphine hydrochloride), eseroline ((-)-eseroline fumarate), ethoxyquin (6-ethoxy-2,2,4-trimethyl-1,2-dihydroquinoline), methyldopa (methyldopa sesquihydrate), olanzapine (2-methyl-4-(4-methyl-1-piperazinyl)-10H-thieno[2,3-b][1,5]benzodiazepine, and indapamide (4-chloro-N-[(2RS)-2-methyl-2,3-dihydro-1H-indol-1-yl]-3-sulfamoyl benzamide).

3. The pharmaceutical composition according to claim 1 or 2 wherein the retinal disease is one or more disease selected from the group consisting of diabetic retinopathy, retinitis pigmentosa, Stargardt disease, hypertensive retinopathy, glaucoma, cataract, central retinal artery occlusion, polypoidal choroidal vasculopathy, Leber congenital amaurosis (LCA), Uscher syndrome, choroideremia, rod-cone or cone-rod dystrophy, ciliary disorder, mitochondrial disease, progressive retinal atrophy, geographic atrophy, familial or acquired macular degeneration, retinal photoreceptor disease, disease on retinal pigment epithelium, cystoid macular edema, retinal degenerative disease, vitreous opacity, uveitis, retinal detachment, traumatic retinal damage, iatrogenic retinal injury, macular hole, macular telangiectasia, ganglion cell disease, optic nerve cell disease, optic neuropathy, ischemic retinal disease, retinopathy of prematurity, retinal vascular occlusion, familial macroaneurysm, retial vascular disease, ocular vascular disease, vascular disease, and ischemic optic neuropathy.

4. The pharmaceutical composition according to any one of claims 1 to 3 wherein the retinal disease is an age-related macular degeneration.

5. The pharmaceutical composition according to any one of claims 1 to 4 wherein the age-related macular degeneration is atrophic (dry) age-related macular degeneration.

6. The pharmaceutical composition according to any one of claims 1 to 4 wherein the age-related macular degeneration is exudative (wet) age-related macular degeneration.

7. The pharmaceutical composition according to any one of claims 1 to 6 which is formulated into eye drop or eye ointment.

8. The pharmaceutical composition according to any one of claims 1 to 7 wherein an effective dose of the compound is a low dose of about one-tenth or less of the half-lethal dose of each compound.

9. The pharmaceutical composition according to any one of claims 1 to 8 wherein an effective dose of the compound is about five(5) weight % or less based on the weight of the pharmaceutical composition for intraocular administration, and is about eighty(80) weight % or less based on the weight of the pharmaceutical composition for oral administration.

10. An eye drop for preventing or treating a retinal disease, or suppressing a progression of the disease in a subject in need of treatment, the eye drop comprising an effective amount of one or more compounds selected from the group described in claim 1 or 2, and a pharmaceutically acceptable carrier.

11. An oral drug for preventing or treating a retinal disease, or suppressing a progression of the disease in a subject in need of treatment, the oral drug comprising an effective amount of one or more compounds selected from the group described in claim 1 or 2, and a pharmaceutically acceptable carrier.

12. Use of one or more compounds selected from the group described in claim 1 or 2 in preparation of a pharmaceutical for intraocular administration or oral administration for preventing or treating a retinal disease, or suppressing a progression of the disease in a subject in need of treatment.

13. A method for preventing or treating a retinal disease, or suppressing a progression of the disease in a subject in need of treatment, which comprises administering an effective amount of one or more compounds selected from the group described in claim 1 or 2 to a subject in need of treatment.


SEQUENCE LISTING

<110> Ken-ichi, YAMADA,
      FUSO PHARMACEUTICAL INDUSTRIES, LTD.

<120> Pharmaceutical composition for intraocular or oral administration for
treatment of retinal disease

<130> 675211

<150> JP 2019-001979
<151> 2019-01-09

<160> 40

<170> PatentIn version 3.5

<210> 1
<211> 20
<212> DNA
<213> Artificial Sequence

<220>
<223> Description of Artificial Sequence: primer

<400> 1
ccttgacatt gagattgcca                                                    20


<210> 2
<211> 20
<212> DNA
<213> Artificial Sequence

<220>
<223> Description of Artificial Sequence: primer

<400> 2
gtatcaacca gagggagtga                                                   20


<210> 3
<211> 18
<212> DNA
<213> Artificial Sequence

<220>
<223> Description of Artificial Sequence: primer

<400> 3
aagacactgt ggctcgtc                                                     18


<210> 4
<211> 18
<212> DNA
<213> Artificial Sequence

<220>
<223> Description of Artificial Sequence: primer

<400> 4
cttcctgtag gtggcaat                                                     18

<210> 5
<211> 23
<212> DNA
<213> Artificial Sequence

<220>
<223> Description of Artificial Sequence: primer

<400> 5
ccaacaagct ggtgctatgt gaa                                              23


<210> 6
<211> 23
<212> DNA
<213> Artificial Sequence

<220>
<223> Description of Artificial Sequence: primer

<400> 6
ctggttgctc accatgtcca tta                                              23


<210> 7
<211> 20
<212> DNA
<213> Artificial Sequence

<220>
<223> Description of Artificial Sequence: primer

<400> 7
aatgtggtat gcgcctctgg                                                  20


<210> 8
<211> 20
<212> DNA
<213> Artificial Sequence

<220>
<223> Description of Artificial Sequence: primer

<400> 8
gcagcaaccc tccaatgaaa                                                  20


<210> 9
<211> 20
<212> DNA
<213> Artificial Sequence

<220>
<223> Description of Artificial Sequence: primer

<400> 9
gtggcacctc catctgagga                                                  20


<210> 10

<211> 20
<212> DNA
<213> Artificial Sequence

<220>
<223> Description of Artificial Sequence: primer

<400> 10
caccatcagc ttggcctgtt                                                    20


<210> 11
<211> 20
<212> DNA
<213> Artificial Sequence

<220>
<223> Description of Artificial Sequence: primer

<400> 11
gtccttgaag cgaatgctgg                                                    20


<210> 12
<211> 20
<212> DNA
<213> Artificial Sequence

<220>
<223> Description of Artificial Sequence: primer

<400> 12
cattctcaag atggcagatc                                                    20


<210> 13
<211> 22
<212> DNA
<213> Artificial Sequence

<220>
<223> Description of Artificial Sequence: primer

<400> 13
ctctctaagg ctacaggctg ct                                                 22


<210> 14
<211> 20
<212> DNA
<213> Artificial Sequence

<220>
<223> Description of Artificial Sequence: primer

<400> 14
tcacggttgc aagagaaaca                                                    20


<210> 15
<211> 21
<212> DNA
<213> Artificial Sequence

<220>
<223> Description of Artificial Sequence: primer

<400> 15
gtgtgactac agcacaagcc g                                                21


<210> 16
<211> 22
<212> DNA
<213> Artificial Sequence

<220>
<223> Description of Artificial Sequence: primer

<400> 16
cccaaggaca tattcacagc ct                                               22


<210> 17
<211> 21
<212> DNA
<213> Artificial Sequence

<220>
<223> Description of Artificial Sequence: primer

<400> 17
tgcagaaacc ctcttggcat c                                                21


<210> 18
<211> 21
<212> DNA
<213> Artificial Sequence

<220>
<223> Description of Artificial Sequence: primer

<400> 18
tgaaacctcc cagctctttc c                                                21


<210> 19
<211> 20
<212> DNA
<213> Artificial Sequence

<220>
<223> Description of Artificial Sequence: primer

<400> 19
tttgatcact gtggggatga                                                  20


<210> 20
<211> 21
<212> DNA
<213> Artificial Sequence

<220>
<223> Description of Artificial Sequence: primer

<400> 20
acactttctg gtgaaggctt g                                                21

<210> 21
<211> 21
<212> DNA
<213> Artificial Sequence

<220>
<223> Description of Artificial Sequence: primer

<400> 21
tggaagtgga tggcatagat g                                                21

<210> 22
<211> 18
<212> DNA
<213> Artificial Sequence

<220>
<223> Description of Artificial Sequence: primer

<400> 22
ttcactgtct gctcaacg                                                    18

<210> 23
<211> 20
<212> DNA
<213> Artificial Sequence

<220>
<223> Description of Artificial Sequence: primer

<400> 23
ccctgtagcc acttctgcaa                                                  20

<210> 24
<211> 20
<212> DNA
<213> Artificial Sequence

<220>
<223> Description of Artificial Sequence: primer

<400> 24
gggcagtgaa cacatctcca                                                  20

<210> 25
<211> 20
<212> DNA
<213> Artificial Sequence

<220>
<223> Description of Artificial Sequence: primer

<400> 25
tctgaagcct ccaacgttcc                                                  20

<210> 26
<211> 20
<212> DNA
<213> Artificial Sequence

<220>
<223> Description of Artificial Sequence: primer

<400> 26
tgggatgggg aaggaaatgc                                                   20


<210> 27
<211> 21
<212> DNA
<213> Artificial Sequence

<220>
<223> Description of Artificial Sequence: primer

<400> 27
gaaaccctgt cactgtcatt c                                                 21


<210> 28
<211> 20
<212> DNA
<213> Artificial Sequence

<220>
<223> Description of Artificial Sequence: primer

<400> 28
ccccaaacac atacacatcc                                                   20


<210> 29
<211> 18
<212> DNA
<213> Artificial Sequence

<220>
<223> Description of Artificial Sequence: primer

<400> 29
ctgggagcgg ctgtatgt                                                     18


<210> 30
<211> 18
<212> DNA
<213> Artificial Sequence

<220>
<223> Description of Artificial Sequence: primer

<400> 30
cacggaagcc atgtaggg                                                     18


<210> 31
<211> 20

<212> DNA
<213> Artificial Sequence

<220>
<223> Description of Artificial Sequence: primer

<400> 31
agcccaacac cagctacatc                                                   20


<210> 32
<211> 20
<212> DNA
<213> Artificial Sequence

<220>
<223> Description of Artificial Sequence: primer

<400> 32
gaatgcccca agttcttcgc                                                   20


<210> 33
<211> 20
<212> DNA
<213> Artificial Sequence

<220>
<223> Description of Artificial Sequence: primer

<400> 33
cagcaaggag gagtcaacat                                                   20


<210> 34
<211> 20
<212> DNA
<213> Artificial Sequence

<220>
<223> Description of Artificial Sequence: primer

<400> 34
tccacaagag cccgtaaatc                                                   20


<210> 35
<211> 20
<212> DNA
<213> Artificial Sequence

<220>
<223> Description of Artificial Sequence: primer

<400> 35
tggtgacaac gactgtggag                                                   20


<210> 36
<211> 21
<212> DNA
<213> Artificial Sequence

<220>
<223> Description of Artificial Sequence: primer

<400> 36
tgttgatccc atagcctgct g                                                21


<210> 37
<211> 17
<212> DNA
<213> Artificial Sequence

<220>
<223> Description of Artificial Sequence: primer

<400> 37
gtgggcatcc cggagac                                                     17


<210> 38
<211> 24
<212> DNA
<213> Artificial Sequence

<220>
<223> Description of Artificial Sequence: primer

<400> 38
caccaaactc aaattgagat ccaa                                             24


<210> 39
<211> 22
<212> DNA
<213> Artificial Sequence

<220>
<223> Description of Artificial Sequence: primer

<400> 39
aatgtgtccg tcgtggatct ga                                               22


<210> 40
<211> 22
<212> DNA
<213> Artificial Sequence

<220>
<223> Description of Artificial Sequence: primer

<400> 40
gatgcctgct tcaccacctt ct                                               22